(19) Europäisches Patentamt
European Patent Office
Office européen des brevets

(11) Publication number: 0 464 656 A1

# (12) EUROPEAN PATENT APPLICATION

(21) Application number: 91110608.6

(22) Date of filing: 26.06.91

(51) Int. Cl.5: C07D 207/12, C12P 17/10, C12N 1/20, //(C12N1/20, C12R1:465)

(30) Priority: 29.06.90 JP 169759/90

(43) Date of publication of application: 08.01.92 Bulletin 92/02

(84) Designated Contracting States: DE FR GB

(71) Applicant: MICROBIAL CHEMISTRY RESEARCH FOUNDATION
14-23, Kamiosaki 3 Chome Shinagawa-ku
Tokyo 141(JP)

Applicant: AJINOMOTO CO., INC.
5-8, Kyobashi 1-chome, Chuo-ku
Tokyo 104(JP)

(72) Inventor: Takeuchi, Tomio
1-11, Higashigotanda 5-chome,
Shinagawa-ku, Tokyo(JP)

Inventor: Okami, Yoshiro
18-14, Denenchofu 4-chome,
Ota-ku, Tokyo(JP)

Inventor: Kurasawa, Shogo c/o Ajimoto Co., Inc.
Central Res. Lab. 1-1, Suzuki-cho,
Kawasaki-ku,
Kawasaki-shi, Kanagawa-ken,(JP)

Inventor: Kameyama, Toshiyuki c/o Ajimoto Co., Inc.
Centr. Res. Lab., 1-1, Suzuki-cho,
Kawasaki-ku,
Kawasaki-shi, Kanagawa-ken(JP)

(74) Representative: Strehl, Schübel-Hopf, Groening
Maximilianstrasse 54 Postfach 22 14 55
W-8000 München 22(DE)

(54) **SA3097 compounds, a process for production thereof and use thereof.**

(57) There is provided SA3097 compounds represented by general formula (1) below,

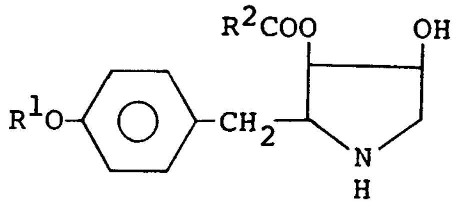

..... (1)

and process for production thereof as well as antitumor agents comprising said compounds as an effective ingredient.

EP 0 464 656 A1

## Field of the invention

The present invention relates to novel SA3097 compounds and a process for production thereof as well as antitumor agents comprising the compounds as effective ingredients.

## Brief description of related art

A known compound, anisomycin may be represented by the formula

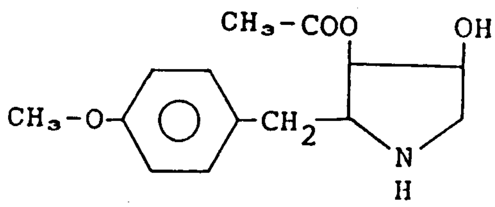

In 1954, this compound was found in Actinomyces as a protozoacidal antibiotic. It is recognized by clinical tests that this compound is effective against amoebic dysentery or trichomonas vaginitis. It is also known that the compound has an effect of inhibiting pathogenic fungi in plants such as mildew in Leguminosae, etc. A growth inhibitory activity against human tumor HeLa cells has also been reported.

However, it is unknown that anisomycin has an antitumor activity.

The present invention relates to SA3097 compounds represented by general formula (1) below:

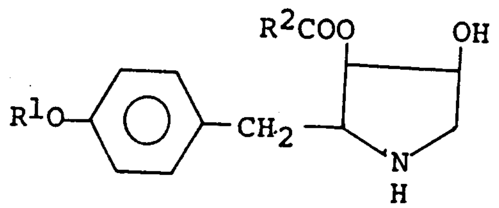

..... (1)

wherein $R^1$ represents hydrogen or a lower alkyl group and $R^2$ represents a lower alkyl group, wherein however $R^1$ and $R^2$ are not simultaneously $CH_3$-groups, and pharmacologically acceptable salts thereof; a process for producing the SA3097 compounds represented by general formula (1) described above, which comprises culturing a microorganism belonging to the genus Streptomyces and capable of producing the SA3097 compounds, and harvesting the compounds from the culture; Streptomyces sp. SA-3097 strain (FERM BP-3380 ) Which is a bacterium capable of producing the SA3097 compounds; and antitumor compositions comprising as an effective ingredient the SA3097 compounds pharmacologically acceptable salts thereof.

The invention is further illustrated by the drawings.

Fig. 1 shows $^1$H-NMR spectrum of SA3097B; Fig. 2 shows $^{13}$C-NMR spectrum thereof; and Fig. 3 shows UV absorption spectrum thereof. Fig. 4 shows $^1$H-NMR spectrum of SA3097$D_2$; Fig. 5 shows $^{13}$C-NMR spectrum thereof; and Fig. 6 shows UV absorption spectrum thereof. Fig. 7 shows $^1$H-NMR spectrum of SA3097$D_1$; and Fig. 8 shows UV absorption spectrum thereof.

Preferred embodiments of this invention are now described in more detail.

The SA3097 compounds of the present invention are represented by general formula (1) described above. In this formula, lower alkyl groups are preferably $C_1$ to $c_6$ alkyl groups. Specific examples include a compound wherein $R^1$ is methyl group and $R^2$ is ethyl group (hereafter referred to as SA3097B), a compound wherein $R^1$ is hydrogen and $R^2$ is methyl group (hereafter referred to as SA3097$D_1$), a compound wherein $R^1$ is hydrogen and $R^2$ is ethyl group (hereafter referred to as SA3097$D_2$). The physicochemical properties of these compounds are shown below.

| | SA3097 | | |
|---|---|---|---|
| | B | $D_1$ | $D_2$ |
| TLC (silica gel) Rf value: | | | |
| Chloroform/methanol (7/3) | 0.62 | 0.24 | 0.37 |
| Chloroform/methanol/ conc. ammonium hydroxide (4/1/0/1) | 0.74 | 0.40 | 0.47 |
| Color forming reaction: | | | |
| Molybdenum-sulfuric acid reaction | positive | positive | positive |
| Chlorine-toluidine reaction | positive | positive | positive |
| Ninhydrin reaction | positive | positive | positive |
| Anisaldehyde reaction | negative | negative | negative |
| Mass spectrum: | | | |
| (FAB(POS)m/z) | 280($MH^+$) | 252($MH^+$) | 266($MH^+$) |
| Molecular formula: | $C_{15}H_{21}NO_4$ | $C_{13}H_{17}NO_4$ | $C_{14}H_{19}NO_4$ |

The compounds described above are not present in known substances and hence, the present inventors determined that these SA3097 compounds are novel substances.

The SA3097 compounds of the present invention can be obtained by culturing a microorganism belonging to the genus Streptomyces and capable of producing the SA3097 compounds, and harvesting the compounds from the culture. As the microorganism belonging to the genus Streptomyces and capable of producing the SA3097 compounds, there is, for example, Streptomyces sp. SA-3097 strain. This strain was isolated by the present inventors from the submarine deposit on the coast of Kanagawa Prefecture, Miura City and has the following bacteriological properties.

The strain belongs to Actinomyces of the genus Streptomyces since L,L-diaminopimelic acid was detected in cell wall. Culture properties and physiological properties of this strain and carbohydrate utilization are shown in the table described below. Description of color in various media follows Color Harmony Manual by Container Corporation of America. All tests on properties were juged after culturing at 27°C for 2 weeks.

Table 1. Culture Property

| Medium | Growth | Color of Aerial Mycelium | Color of Colony Reverse | Formation of Soluble Pigment |
|---|---|---|---|---|
| Sucrose-nitrate agar medium | good | light olive grey | greyish white | negative |
| Glucose-asparagine agar medium | good | yellowish grey | yellowish grey | negative |
| Yeast-maltose agar medium | abundant | light olive grey | light dull yellow | negative |
| Oatmeal agar medium | good | yellowish grey | yellowish grey | negative |
| Starch-inorganic salts agar medium | abundant | light olive grey | light olive grey | negative |
| Glycerine-asparagine agar medium | weak | none | light brown grey | negative |
| Peptone-yeast extract agar medium | abundant | light olive grey | light olive | negative |
| Tyrosine agar medium | good | greyish white | brownish grey | negative |
| Nutreint agar medium | abundant | light olive grey | light yellowish brown | negative |

Table 2. Physiological Properties

| | |
|---|---|
| Liquefaction of gelatin | positive |
| Hydrolysis of starch | positive |
| Hydrolysis of casein | positive |
| Hydrolysis of urea | positive |
| Hydrolysis of esculine | negative |
| Tyrosinase activity | negative |
| Reduction of nitrate | positive |
| Peptonization of defatted milk | positive |
| Solidification of defatted milk | positive |
| Production of hydrogen sulfide | negative |
| Resistant to saline concentration | 6% |
| Growth temperature | 15.0-43.2°C |
| Optimum temperature | 21.5-32.2°C |

Table 3.

| Carbohydrate Utilization | |
|---|---|
| D-Glucose | positive |
| D-Fructose | positive |
| L-Arabinose | positive |
| D-Xylose | pseudo positive |
| L-Ramnose | negative |
| Sucrose | negative |
| Raffinose | negative |
| D-Mannitol | negative |
| Inositol | negative |

The present strain having the properties described above has been deposited at the Fermentation Research Institute, Agency of Industrial Science & Technology, Japan and its accession number is FERM P-11481 (FERM BP-3380). This invention also embraces mutations of the strain obtained by artificial or spontaneous mutation, which are usable in the present invention so long as they are capable of producing the SA3097 compounds described above.

The microorganism capable of producing the SA3097 compound is cultured in a medium containing carbon sources, nitrogen sources, inorganic ions and, if necessary, nutrients such as vitamins, amino acids, etc. Herein, as the carbon sources, any carbon sources ordinarily used for culture of microorganisms may be optionally used; for example, glucose, sucrose, glycerol, etc. may be used. As the nitrogen sources, any nitrogen sources ordinarily used for culture of microorganisms may similarly be used; there are, for example, peptone, yeast extract, meat extract, corn steep liquor, soybean powders, casein, ammonium ions, etc.

Culture may be carried out under such conditions that the microorganism capable of producing the

SA3097 compounds can grow. The SA3097 compounds are produced by, e.g., liquid aerobic spinner culture. The culture temperature is generally between 10 and 35 °C, preferably between 24 and 30 °C. The culture may be continued until the desired SA3097 compounds are sufficiently accumulated in the culture medium and it is generally for 16 hours to 5 days.

To harvest and purify the SA3097 compounds from the culture medium, known techniques may apply. For example, the culture medium is subjected to solid-liquid separation by centrifugation etc. and the resulting supernatant is passed through a synthetic adsorption resin; the obtained active fraction is concentrated by an appropriate means and the concentrate is passed through a silica gel column and developed with a suitable solvent. The active fraction is collected and concentrated, etc.

According to the present invention, there are provided novel SA3097 compounds and pharmacologically acceptable salts thereof, a process for producing these compounds, the antitumor compositions comprising as effective ingredients the compounds or pharmacologically acceptable salts thereof.

Next, the present invention is described in more detail by referring to the examples.

Example 1

Streptomyces sp. SA-3097 strain (FERM P-11481, FERM BP-3380) was inoculated on 10 liters of 25% artificial seawater (manufactured by German Laboratories, Jamarin S (pH 7.0) containing 20 g of glycerol, 10 g of Bacto-soyton (manufactured by Difco) followed by jar fermentor at 27 °C for 4 days.

After completion of the culture, the cells were removed by suction filtration and 9.4 liters of the culture supernatant was passed through 500 ml of synthetic adsorption resin (HP-20, manufactured by Mitsubishi Chemical Industry Co., Ltd.). The adsorbed fraction was eluted with 2 liters of 0.05N HCl/acetone (1:1). The eluate was concentrated up to 200 ml with an evaporator under reduced pressure. The concentrate was filled up in 100 ml of synthetic adsorption resin (CHP-20, manufactured by Mitsubishi Chemical Industry Co., Ltd.). Gradient elution was carried out with 500 ml of 0.01N HCl and 500 ml of acetone/0.01N HCl (2:8) and fractionation was performed by 10 ml each. The active fraction was collected and concentrated to dryness. After the residue was again dissolved in methanol, the solution was passed through a silica gel column. By developing with chloroform/methanol (7:3), the active fraction was collected and concentrated to dryness. After the residue was again dissolved in methanol, the solution was charged in a silica gel TLC plate of 20 cm x 20 cm. By developing with chloroform/methanol (8:2), the product was isolated by scraping. It was confirmed by $^{1}$H-NMR (conditions for measurement: 4 mg/0.5 ml methanol-d4) (Fig. 1), $^{13}$C-NMR (conditions for measurement: 4 mg/0.5 ml methanol-d4) (Fig. 2), mass spectrum and UV absorption spectrum (solvent: methanol) (Fig. 3) that this compound was SA3097B.

Example 2

Streptomyces sp. SA-3097 strain (FEPM P-11481, FERM BP-3380) was inoculated on 10 liters of medium having the same composition as in Example 1 followed by jar fermentor culture at 27 °C for 4 days.

After completion of the culture, the active fraction was obtained in a manner similar to Example 1 and concentrated to dryness. After the residue was again dissolved in methanol, the solution was passed through a silica gel column. By developing with chloroform/methanol (7:3), the fraction containing an active substance showing Rf value of 0.2 or less when developed by TLC (solvent: chloroform/methanol (8:2)) was collected and concentrated to dryness. After the residue was again dissolved in methanol, the solution was charged in a silica gel TLC plate of 20 cm x 20 cm. By developing with chloroform/methanol/ammonia (20:5:0.5), the product was isolated by scraping.

It was confirmed by $^{1}$H-NMR (conditions for measurement: 6 mg/0.5 ml methanol-d4) (Fig. 4), $^{13}$C-NMR (conditions for measurement: 6 mg/0.5 ml methanol-d4) (Fig. 5), mass spectrum and UV absorption spectrum (solvent: methanol) (Fig. 6) that this compound was SA3097$D_2$.

Example 3

Streptomyces sp. SA-3097 strain (FERM P-11481, FEPM BP-3380) was inoculated on 10 liters of medium having the same composition as in Example 1 followed by jar fermentor culture at 27 °C for 4 days.

After completion of the culture, a compound was isolated by the same procedures as in Example 2.

It was confirmed by $^{1}$H-NMR (conditions for measurement: 6 mg/0.5 ml methanol-d4) (Fig. 7) and, mass spectrum and UV absorption spectrum (solvent: methanol) (Fig. 8) that this compound was SA3097$D_1$.

Example 4
Effect of inhibiting growth against human tumor cells

The effect of inhibiting growth against human lung tumor cell (LU99) and human mammary tumor cell (MCF-7) was determined in vitro. The cells were suspended in RPMI 1640 medium containing 10% fetal calf serum in 2 x 10/ml. The cell suspension was inoculated on a 96 well microplate in 100 µl/well each followed by culturing at 37 °C for a day. After 10 µl of a sample was added, culture was continued for further 3 days. Then, the cells were counted by reduction with MTT reagent (dimethylthiazolyldiphenyl tetrazolium). The sample used was prepared by suspending 2 mg/ml of the test compound in methanol and then diluting with medium. In Table 4 below, 50% growth inhibition concentration is shown.

Table 4

| 50% Inhibition Concentration (M) | | |
|---|---|---|
| Sample | LU99 | MCF-7 |
| SA3097B | $4.1 \times 10^{-8}$ | $5.7 \times 10^{-8}$ |
| SA3097$D_1$ | $8.5 \times 10^{-8}$ | $12.9 \times 10^{-8}$ |
| SA3097$D_2$ | $5.0 \times 10^{-8}$ | $9.5 \times 10^{-8}$ |

Example 5
Activity of inhibiting colony formation in human tumor cells

Using human lung tumor cell (LX-1) and human gastric tumor cell (SC-6), an activity of inhibiting colony formation in soft agar was determined. The cells were treated with a sample for an hour and then washed by centrifugation. Then, the cells were suspended in soft agar. The cell suspension was inoculated on a plate. After culturing, the number of colonies formed was counted. In Table 5, 50% inhibition concentration of colony formation is shown.

Table 5

| 50% Inhibition Concentration (M) | | |
|---|---|---|
| Sample | LX-1 | SC-6 |
| SA3097$D_1$ + $D_2$ | $2.2 \times 10^{-6}$ | $4.5 \times 10^{-6}$ |

**Claims**

**1.** An SA3097 compound represented by general formula (1) below:

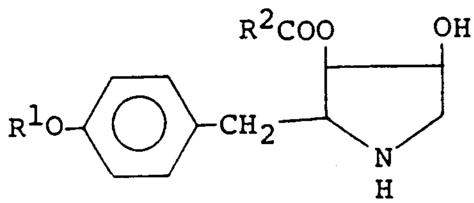

..... (1)

wherein $R^1$ represents hydrogen or a lower alkyl group and $R^2$ represents a lower alkyl group except the case that both $R^1$ and $R^2$ are methyl groups, and a pharmacologically acceptable salt thereof.

**2.** A compound or a pharmacologically acceptable salt thereof as claimed in claim 1, wherein said compound represented by general formula (1) is:

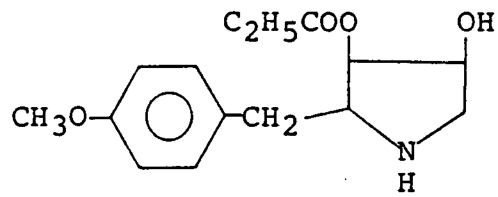

3. A compound or a pharmacologically acceptable salt thereof as claimed in claim 1, wherein said compound represented by general formula (1) is:

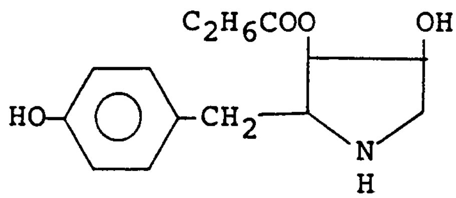

4. A compound or a pharmacologically acceptable salt thereof as claimed in claim 1, wherein said compound represented by general formula (1) is:

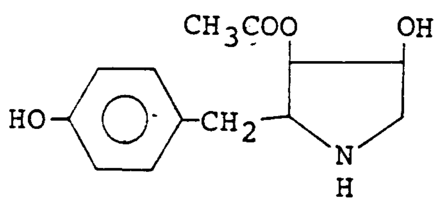

5. A process for producing an SA3097 compound represented by general formula (1) below:

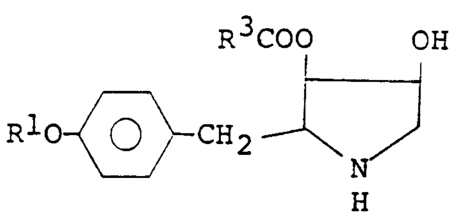

..... (1)

wherein $R^1$ represents hydrogen or a lower alkyl group and $R^2$ represents a lower alkyl group, which comprises culturing a microorganism belonging to the genus Streptomyces and capable of producing said SA3097 compound, and harvesting said compound from the culture.

6. Streptomyces sp. SA-3097 strain (FERM BP-3380) capable of producing an SA3097 compound represented by general formula (1) below:

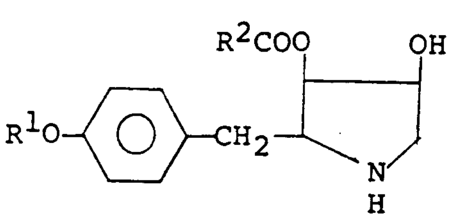

..... (1)

wherein $R^1$ represents hydrogen or a lower alkyl group and $R^2$ represents a lower alkyl group.

**7.** An antitumor composition comprising as an effective ingredient an SA3097 compound represented by general formula (1) below:

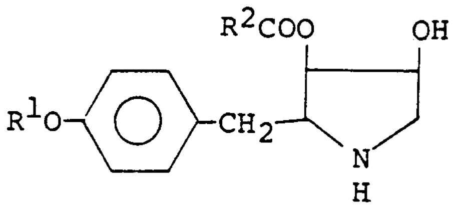

..... (1)

wherein $R^1$ represents hydrogen or a lower alkyl group and $R^2$ represents a lower alkyl group, and a pharmacologically acceptable salt thereof.

FIG.1

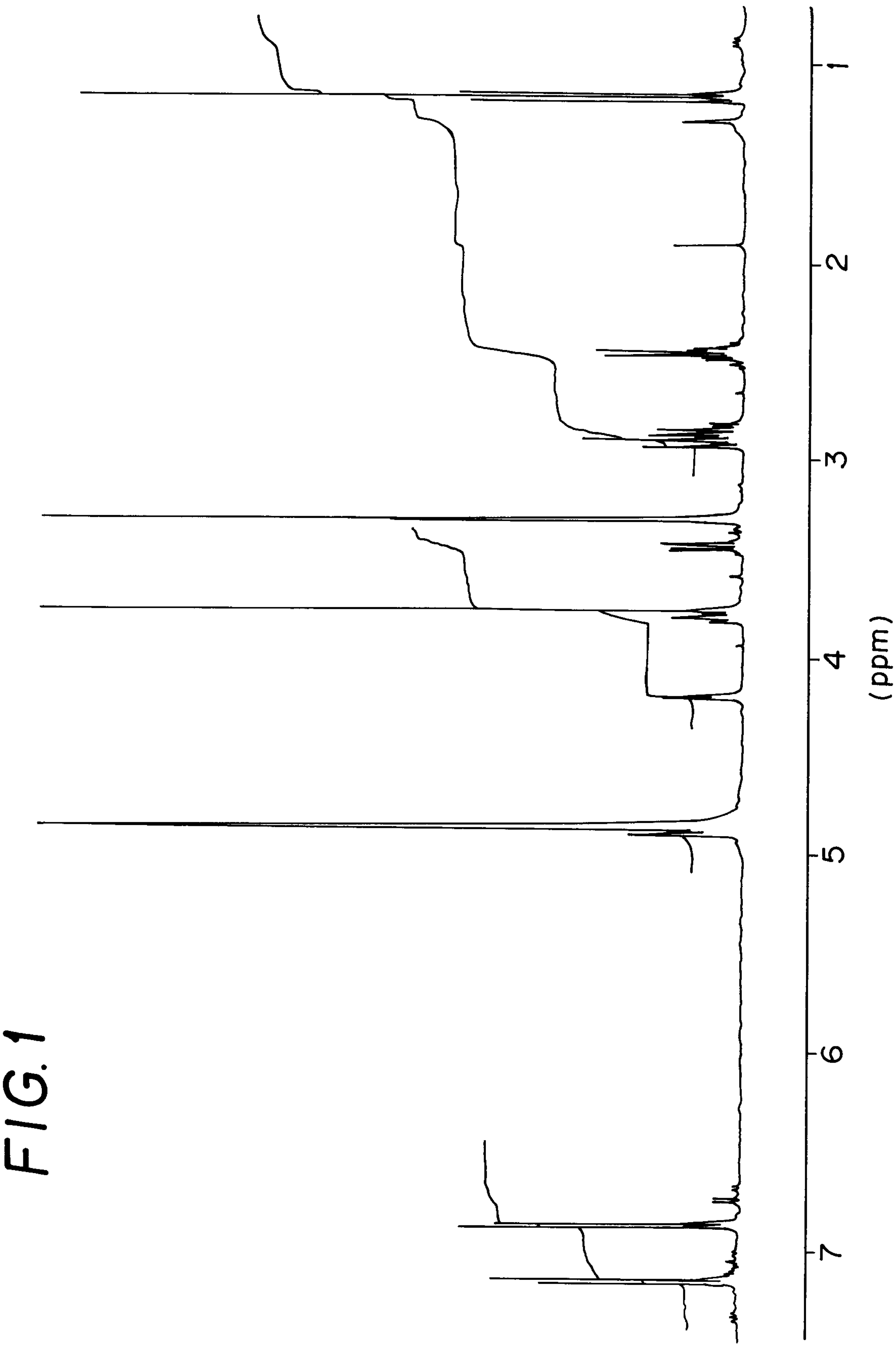

FIG.2

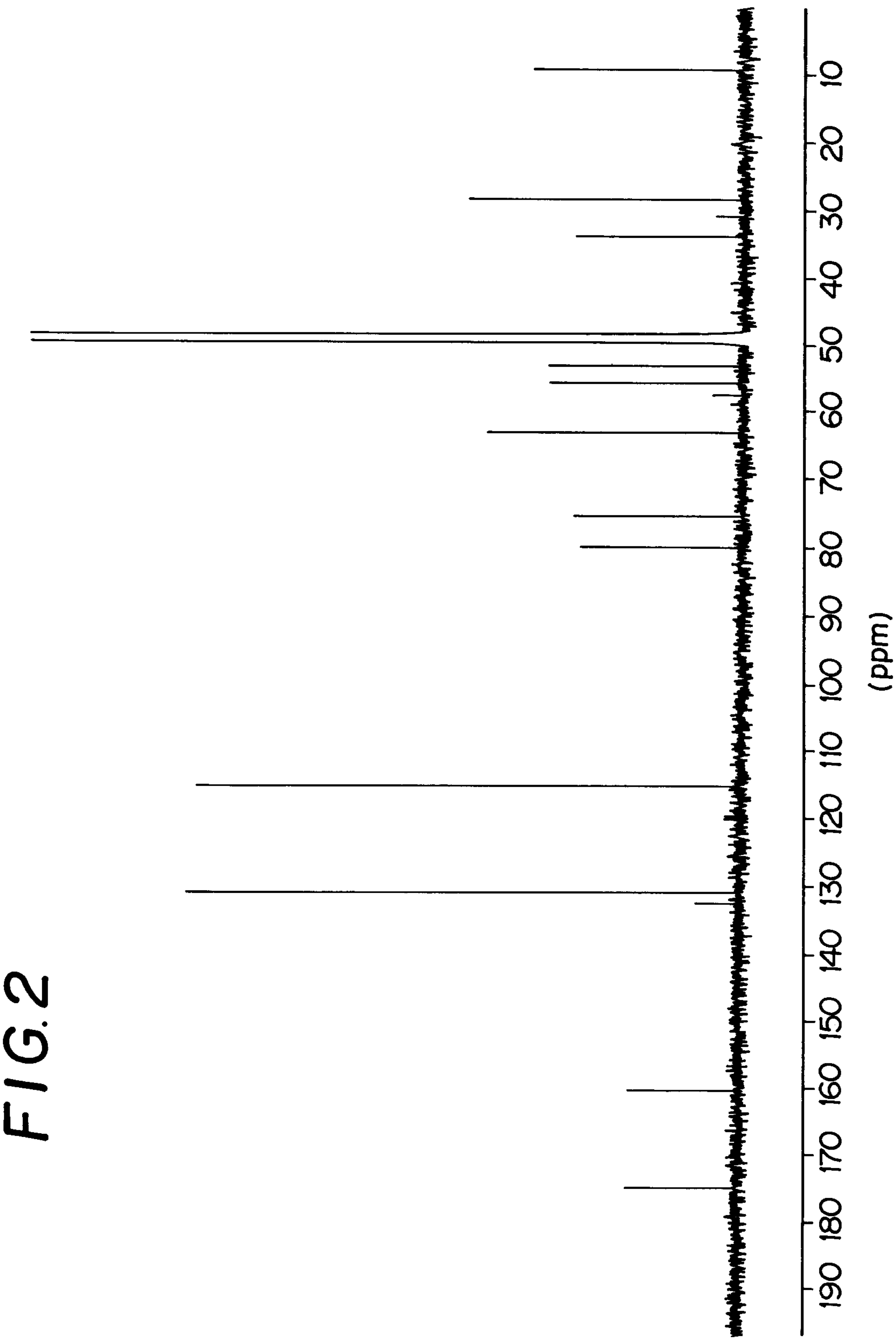
10
20
30
40
50
60
70
80
90
100
110
120
130
140
150
160
170
180
190
(ppm)

FIG. 3

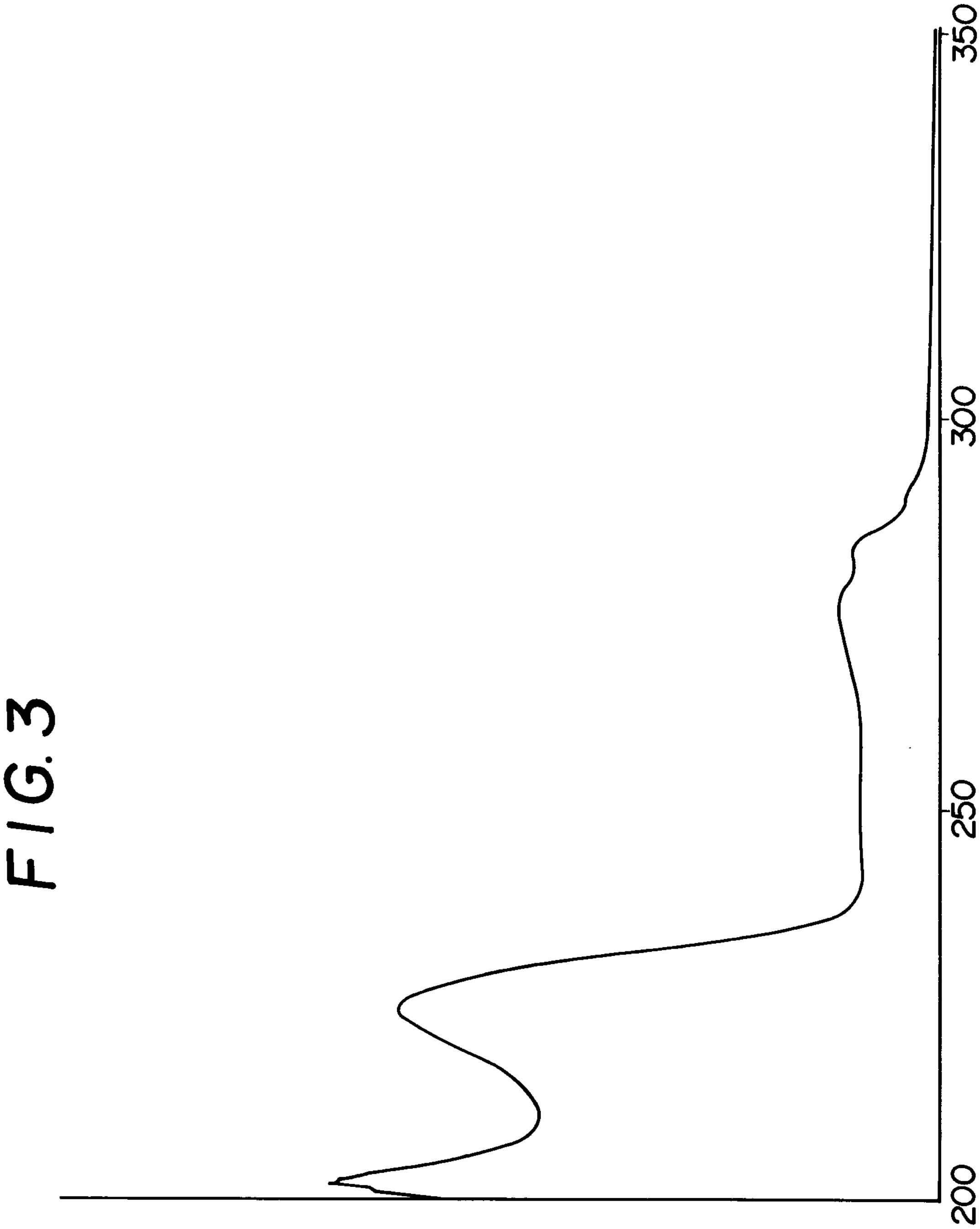

FIG.4

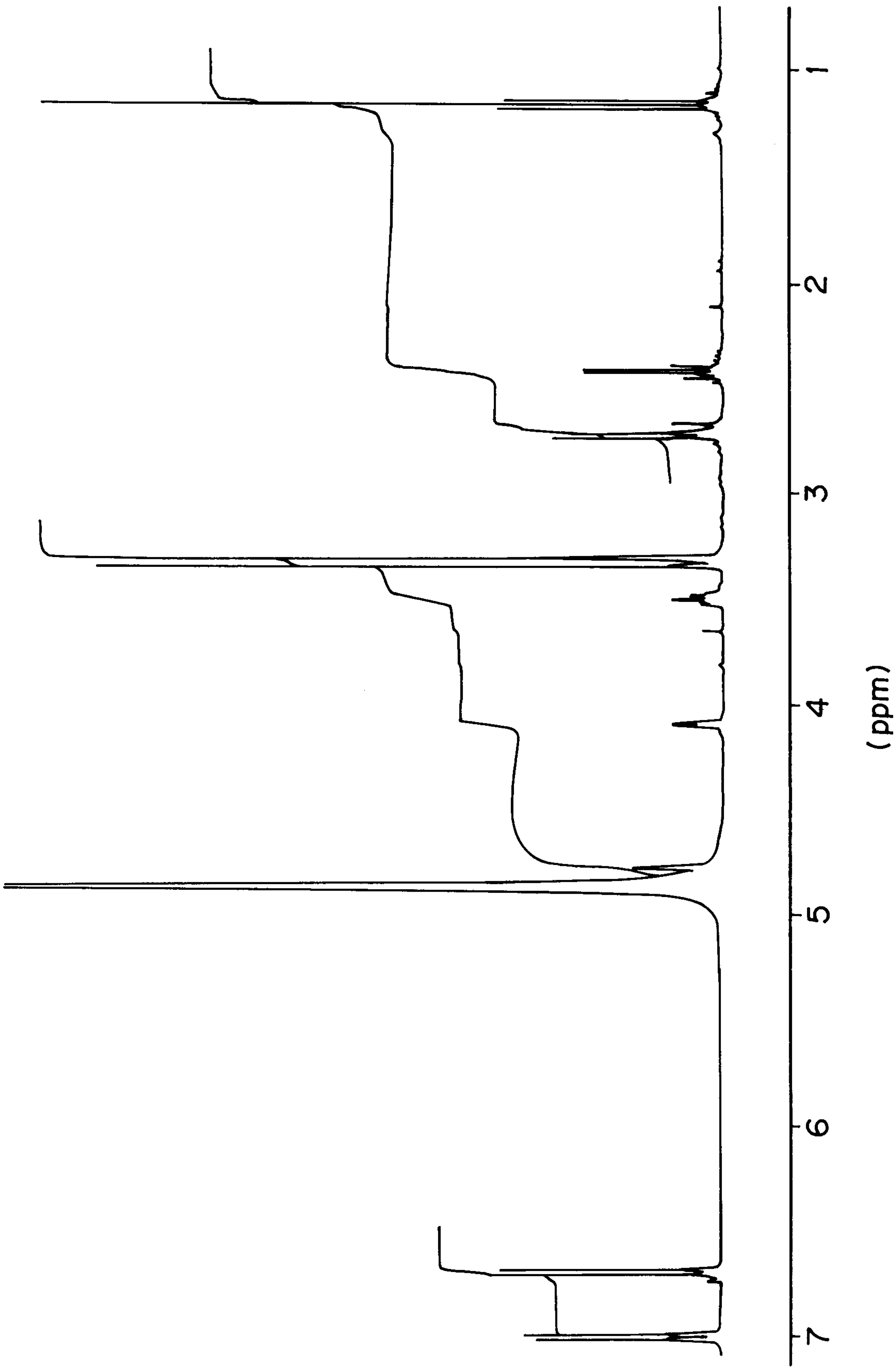

FIG. 5

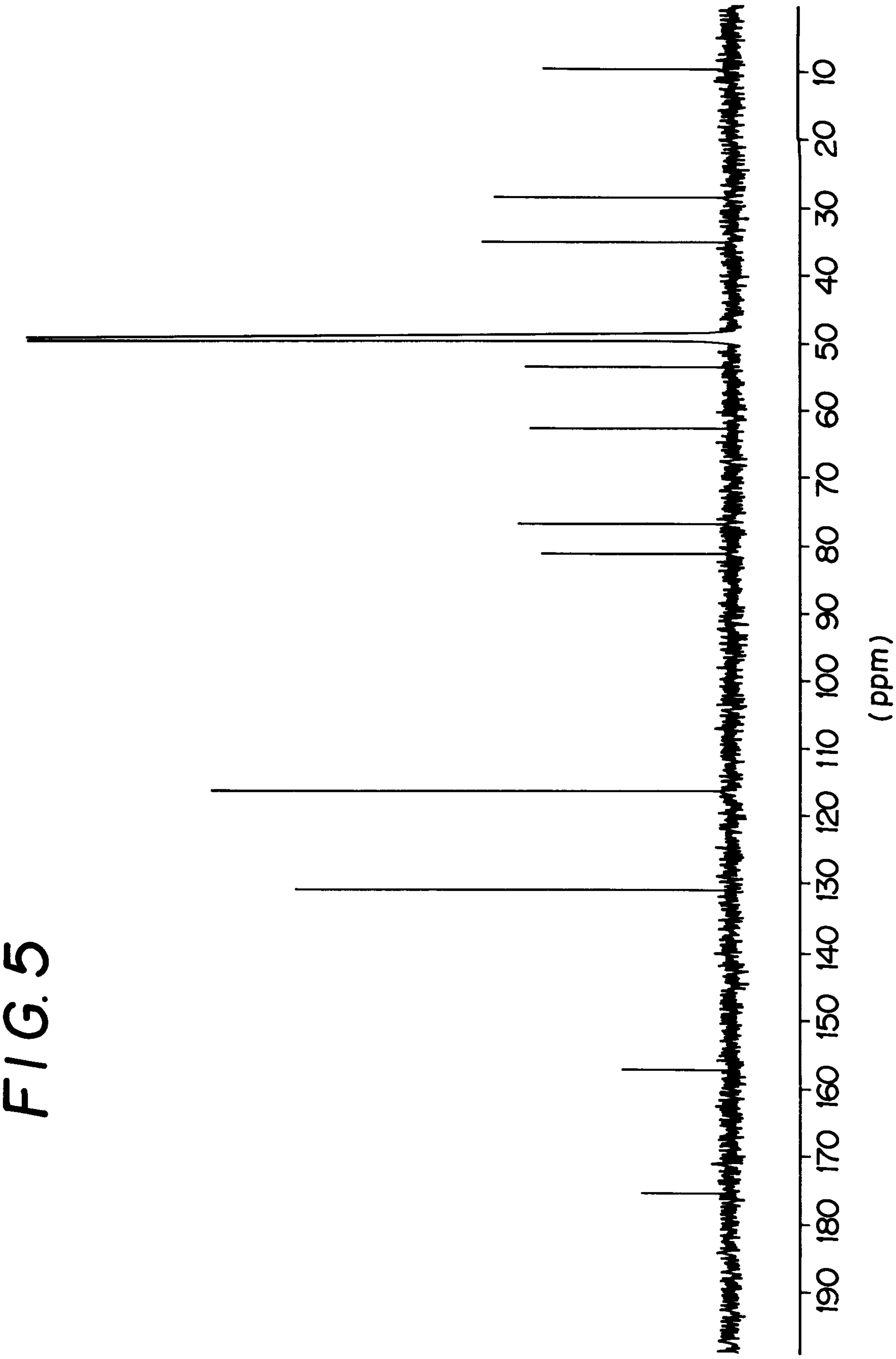
10
20
30
40
50
60
70
80
90
100
110
120
130
140
150
160
170
180
190
(ppm)

FIG. 6

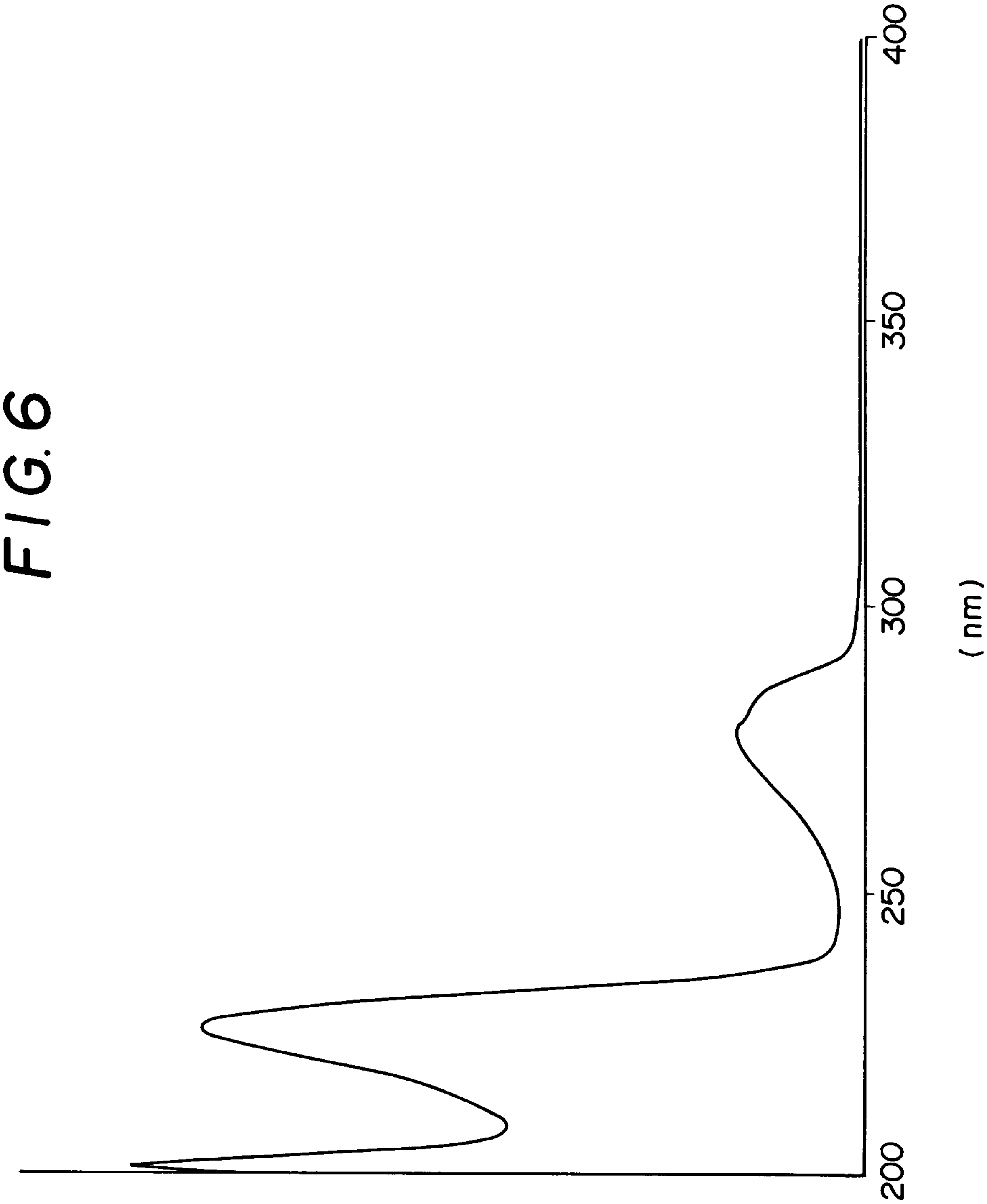

FIG. 7

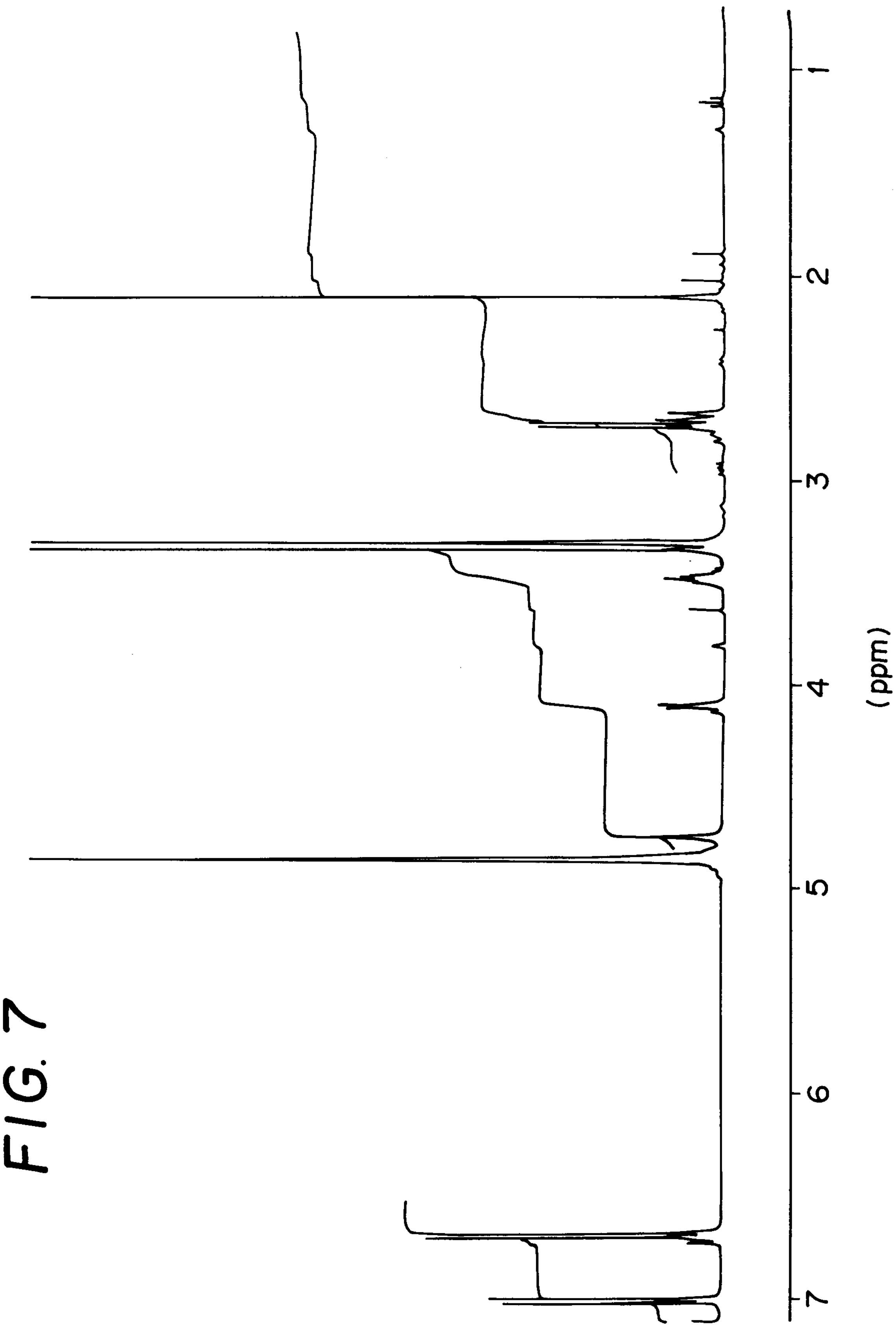

FIG. 8

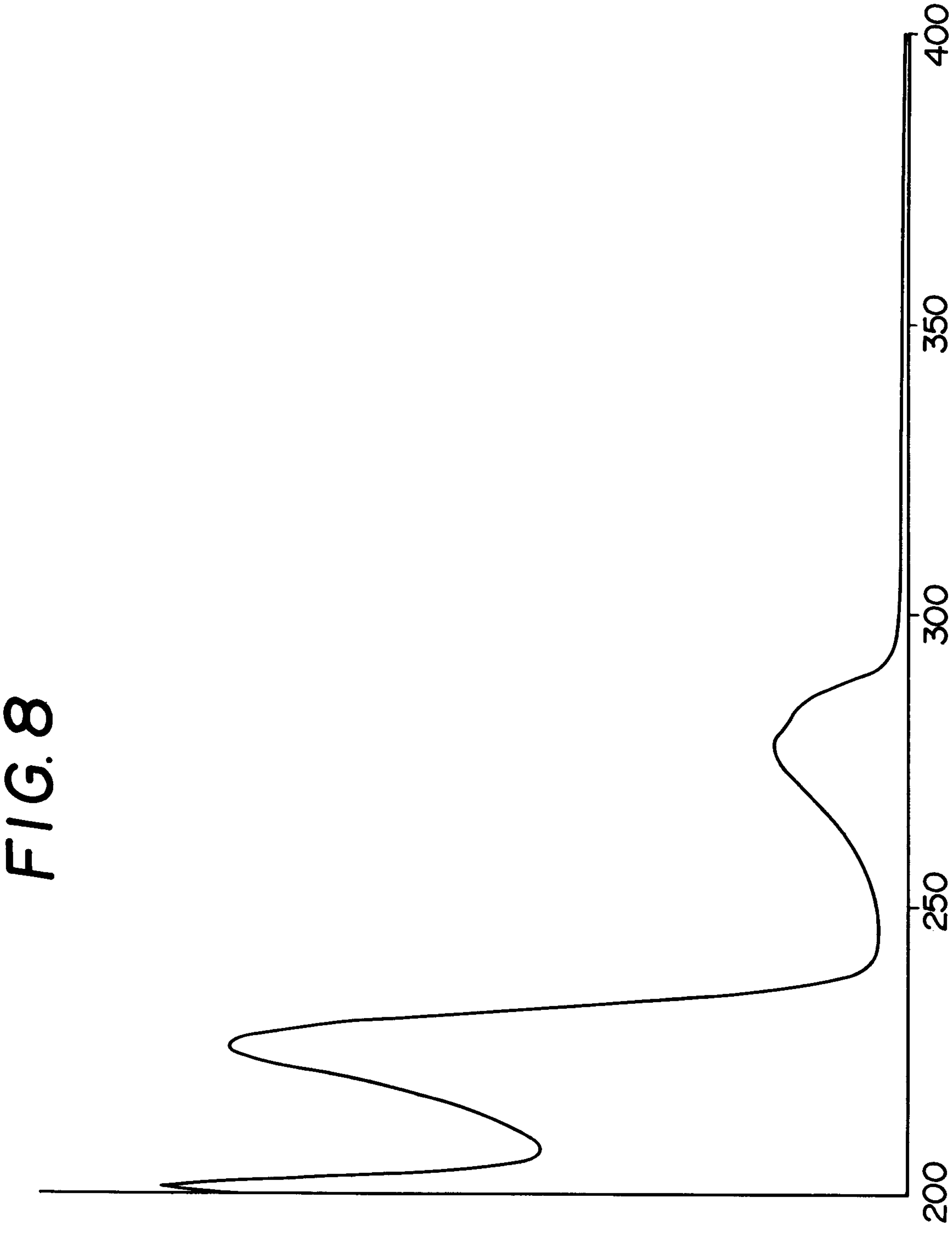

European Patent Office

# EUROPEAN SEARCH REPORT

Application Number

EP 91110608.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 107, No. 25, December 21, 1987, Columbus, Ohio, USA TAGAKI H. et al. "A process for the preparation of O-substituted anisomycins" page 75b, column 1, abstract-No. 236 368c & JP-A2-87/89 659 -- | 1,7 | C 07 D 207/12<br>C 12 P 17/10<br>C 12 N 1/20<br>//(C 12 N 1/20<br>C 12 R 1:465) |
| A | CHEMICAL ABSTRACTS, vol. 98, April 11, 1983, Columbus, Ohio, USA HALL S.S. et al. "Structure-activity relation-ships of synthetic antibiotic analogs of anisomycin" page 20, column 1, abstract-No. 119 143n & J.Med.Chem., 26(4),469-75 (1983) -- | 1 | |
| A | CHEMICAL ABSTRACTS, vol. 63, No. 5, August 30, 1965, Columbus, Ohio, USA BEEREBOOM J.J. et al. "Anisomycin" column 5582, abstract-No. 5582g & J.Org.Chem., 30(7),2334-42 (1965) ---- | 1,5,6 | |

TECHNICAL FIELDS SEARCHED (Int. Cl.5)

C 07 D 207/00
C 12 P 17/00
C 12 N 1/00

The present search report has been drawn up for all claims

| Place of search | Date of completion of the search | Examiner |
|---|---|---|
| VIENNA | 30-08-1991 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document